# EUROPEAN PATENT APPLICATION

(11) **EP 3 992 277 A1**
(43) Date of publication of application: **04.05.2022**
(21) Application number: 20833264.3
(22) Date of filing: 23.06.2020
(51) Int. Cl.: C12M 3/00

(54) **CELL CULTURING VESSEL AND CELL CULTURING APPARATUS**

(30) Priority: 28.06.2019 US 201962868532 P
(71) Applicant: I Peace, Inc., Palo Alto, CA 94303 (US); FANUC CORPORATION, Oshino-mura Minamitsuru-gun Yamanashi 401-0597 (JP)
(72) Inventor: TANABE, Koji, Palo Alto, California 94303 (US); HIRAIDE, Ryoji, Kyoto-shi, Kyoto 615-8245 (JP); BAN, Kazunori, Minamitsuru-gun, Yamanashi 401-0597 (JP); KINOSHITA, Satoshi, Minamitsuru-gun, Yamanashi 401-0597 (JP)
(74) Representative: MacLean, Martin Robert
(86) International application number: PCT/JP2020/024548
(87) International publication number: WO 2020/262354

(57) **Abstract**

There is provided a cell culture vessel for culturing a cell in an interior thereof, wherein a width of a bottom surface is equal to or larger than a height of a side surface, the cell culture vessel comprising a flow path configured to supply a fluid into the interior, and wherein the interior is able to be closed.

## Description

### Technical Field

The present invention relates to a cell technology, and relates to a cell culture vessel and a cell culture device.

### Background Art

Embryonic stem cells (ES cells) are stem cells derived from early human or mouse embryos. ES cells have pluripotency that allows them to differentiate into any type of cells in a living body. Currently, human ES cells can be used for cell transplantation therapy for numerous diseases such as Parkinson's disease, juvenile diabetes, and leukemia. However, there are also obstacles to ES cell transplantation. In particular, ES cell transplantation can elicit immunorejection similar to a rejection response that follows unsuccessful organ transplantation. In addition, there are many criticisms and dissenting opinions from a moral point of view regarding use of ES cells derived by destroying human embryos.

Under such circumstances, Professor Shinya Yamanaka at Kyoto University succeeded in deriving induced pluripotent stem cells (iPS cells) by introducing four genes: OCT3/4, KLF4, c-MYC, and SOX2 into somatic cells. For this, Professor Yamanaka was awarded the 2012 Nobel Prize in Physiology or Medicine (for example, refer to Patent Documents 1 and 2). iPS cells are ideal pluripotent cells without rejection responses or moral issues. Therefore, iPS cells are expected to be used for cell transplantation therapy.

### Citation List

### Patent Document

Patent Document 1: Japanese Patent No. 4183742
Patent Document 2: Patent Publication JP-A-2014-114997

### Summary

### Technical Problem

A device that can efficiently culture not only iPS cells but also various cells is desired. Therefore, one objective of the present invention is to provide a cell culture vessel and a cell culture device.

### Solution to Problem

According to an aspect of the present invention, there is provided a cell culture vessel for culturing a cell in an interior thereof, wherein a width of a bottom surface is equal to or larger than a height of a side surface, the cell culture vessel including a flow path configured to supply a fluid into the interior, and wherein the interior is able to be closed.

In the cell culture vessel, at least one of a bottom surface and a top surface may be transparent.

According to an aspect of the present invention, there is provided a cell culture vessel for culturing a cell in an interior thereof, wherein at least one of a bottom surface and a top surface is transparent, the cell culture vessel including a flow path configured to supply a fluid into the interior, and wherein the interior is able to be closed.

The cell culture vessel may include a first housing having the bottom surface; and a second housing which is disposed on the first housing and has a top surface that faces the bottom surface, and the first housing and the second housing may be combined to form the interior.

In the cell culture vessel, the flow path may be provided at at least one of the first housing and the second housing.

The cell culture vessel may further include a temperature adjuster configured to adjust a temperature in the cell culture vessel.

In the cell culture vessel, an internal culture container may be able to be disposed in the interior, and a fluid may be supplied into the internal culture container through the flow path.

The cell culture vessel may further include a medium component permeable member that is disposed in the interior.

In addition, according to an aspect of the present invention, there is provided a cell culture device including: a cell culture vessel for culturing a cell in an interior thereof; and a variable volume container that is connected to the cell culture vessel, wherein a width of a bottom surface of the cell culture vessel is equal to or larger than a height of a side surface, wherein the cell culture vessel includes a flow path configured to supply a fluid into the interior, wherein the variable volume container is connected to the cell culture vessel via the flow path, wherein the fluid is movable in the cell culture vessel and the variable volume container, and wherein the interior of the cell culture vessel and the variable volume container is able to be closed.

In addition, according to an aspect of the present invention, there is provided a cell culture device including: a cell culture vessel for culturing a cell in an interior thereof; and a variable volume container that is connected to the cell culture vessel, wherein at least one of a bottom surface and a top surface is transparent, wherein the cell culture vessel includes a flow path configured to supply a fluid into the interior, wherein the variable volume container is connected to the cell culture vessel via the flow path, wherein the fluid is movable in the cell culture vessel and the variable volume container, and wherein the interior of the cell culture vessel and the variable volume container is able to be closed.

In the cell culture device, the variable volume container may be configured to hold a substance, and the substance may come into contact with the cell according to movement of the fluid.

The cell culture device may further include a flow path configured to supply the cell into the cell culture vessel.

The cell culture device may further include a fluid machine for supplying the cell into the cell culture vessel.

The cell culture device may further include a flow path configured to supply a medium into the cell culture vessel.

The cell culture device may further include a flow path configured to supply a cell dissociation reagent into the cell culture vessel.

The cell culture device may further include a flow path configured to discharge at least part of cells detached from an inner surface of the cell culture vessel with the cell dissociation reagent to the outside of the cell culture vessel.

In the cell culture device, at least part of cells detached from the inner surface of the cell culture vessel with the cell dissociation reagent may be returned to the cell culture vessel.

The cell culture device may further include a flow path configured to supply a cell cryopreservation solution into the cell culture vessel.

The cell culture device may further include a temperature adjuster configured to adjust a temperature in the cell culture vessel.

In the cell culture device, an internal culture container may be able to be disposed in the interior of the cell culture vessel, and a medium may be supplied into the internal culture container.

The cell culture device may further include a medium component permeable member that is disposed in the interior of the cell culture vessel.

### Advantageous Effects of Invention

According to the present invention, it is possible to provide a cell culture vessel and a cell culture device.

### Brief Description of Drawings

Fig. 1 is a schematic front view of a cell culture system according to an embodiment.
Fig. 2 is a schematic perspective view of the cell culture system according to the embodiment.
Fig. 3 is a schematic view of a mononuclear cell collector according to an embodiment.
Fig. 4 is a schematic cross-sectional view of a cell culture vessel according to an embodiment.
Fig. 5 is a schematic cross-sectional view of the cell culture vessel according to the embodiment.
Fig. 6 is a schematic cross-sectional view of the cell culture vessel according to the embodiment.
Fig. 7 is a schematic cross-sectional view of the cell culture vessel according to the embodiment.
Fig. 8 is a schematic front view of the cell culture system according to the embodiment.
Fig. 9 is a schematic perspective view of the cell culture system according to the embodiment.
Fig. 10 shows a microscopic image of cell masses according to Example 1.
Fig. 11 is a histogram showing the results of flow cytometry of iPS cells according to Example 1.
Fig. 12 shows the analysis results of fluorescence-activated cell sorting according to Example 2.
Fig. 13(a) shows a microscopic image of treated blood before it is put into a mononuclear cell collector according to Example 2 and Fig. 13(b) shows a microscopic image of a solution containing mononuclear cells collected from the mononuclear cell collector.
Fig. 14 is a graph showing the number of platelets in treated blood before it is put into the mononuclear cell collector according to Example 2 and the number of platelets in a solution containing mononuclear cells collected from the mononuclear cell collector.
Fig. 15(a) shows an image of a culture solution containing treated blood containing platelets before it is put into the mononuclear cell collector according to Example 2 and Fig. 15(b) shows an image of a culture solution containing a solution containing mononuclear cells from which platelets are removed.
Fig. 16 shows a microscopic image of cells produced by a method of producing iPS cells according to Example 3.
Fig. 17 is a histogram showing the results obtained by analyzing the cells produced by the method of producing iPS cells according to Example 3 with flow cytometry.
Fig. 18 shows a microscopic image of cells produced by a method of producing iPS cells according to Example 4.
Fig. 19 is a histogram showing the results obtained by analyzing the cells produced by the method of producing iPS cells according to Example 4 with flow cytometry.
Fig. 20 shows a microscopic image of cells produced by a method of producing iPS cells according to Example 5.
Fig. 21 is a histogram showing the results obtained by analyzing the cells produced by the method of producing iPS cells according to Example 5 with flow cytometry.
Fig. 22 shows a microscopic image of cells produced by a method of producing iPS cells according to Example 6.
Fig. 23 is a histogram showing the results obtained by analyzing the cells produced by the method of producing iPS cells according to Example 6 with flow cytometry.

### Description of Embodiments

Hereinafter, embodiments of the present invention will be described. In the following description of the drawings, the same or similar parts are denoted with the same or similar reference numerals. However, the drawings are schematic. Therefore, specific sizes and the like should be determined in light of the following description. In addition, it goes without saying that the drawings include parts having different relationships and ratios with each other.

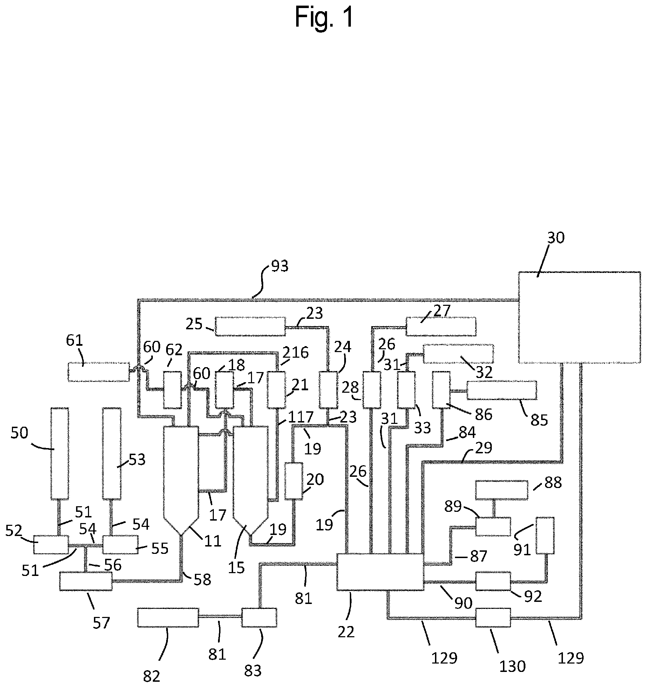

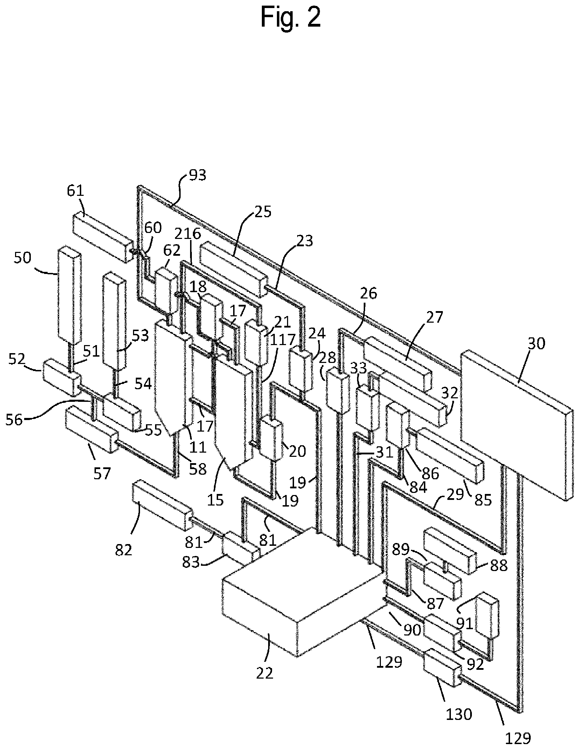

As shown in Fig. 1 and Fig. 2, a cell culture device according to an embodiment includes a cell culture vessel 22 for culturing a cell in an interior and a variable volume container 27 connected to the cell culture vessel 22. For example, the width of the bottom surface of the cell culture vessel 22 is equal to or larger than the height of the side surface. Here, the bottom surface is a surface substantially perpendicular to the direction of gravity. The cell culture vessel 22 includes a flow path 26 configured to supply a fluid into the interior thereof, the variable volume container 27 is connected to the cell culture vessel 22 via the flow path 26, and the fluid is movable in the cell culture vessel 22 and the variable volume container 27. For example, the flow path 26 is connected to the side wall of the cell culture vessel 22. A valve other than the fluid machine may not be provided at the flow path 26. In addition, the interior of the cell culture vessel 22 and the variable volume container 27 can be closed. Here, in the present disclosure, "fluid" may refer to a gas or a liquid.

The cell culture device according to the embodiment includes a blood container 50 configured to hold blood and a red blood cell treatment agent container 53 configured to hold a red blood cell precipitating agent or a red blood cell removal agent.

The interior of the blood container 50 holds the blood. The blood container 50 may have a structure in which the interior can be closed from the outside air. The closed space including the interior of the blood container 50 may be configured such that gases, viruses, microorganisms, impurities and the like are not exchanged with the outside. The blood container 50 may be embedded and enclosed in a non-gas-permeable substance. At least a part of the blood container 50 may be formed by inscribing in a member. At least a part of the blood container 50 may be formed by overlaying recesses inscribed in members. The blood container 50 may be capable of undergoing a change in the volume of the blood container 50. In this case, for example, the blood container 50 includes a syringe that holds a fluid and a plunger which is inserted into the syringe and movable in the syringe, and the volume within the syringe that can hold the fluid can be changed by moving the plunger. Alternatively, the blood container 50 may be a flexible bellows or bag.

The interior of the red blood cell treatment agent container 53 holds the red blood cell precipitating agent or the red blood cell removal agent. The red blood cell treatment agent container 53 may have a structure in which the interior can be closed from the outside air. The closed space including the interior of the red blood cell treatment agent container 53 may be configured such that gases, viruses, microorganisms, impurities and the like are not exchanged with the outside. The red blood cell treatment agent container 53 may be embedded and enclosed in a non-gas-permeable substance. At least a part of the red blood cell treatment agent container 53 may be formed by inscribing in a member. At least a part of the red blood cell treatment agent container 53 may be formed by overlaying recesses inscribed in members. The red blood cell treatment agent container 53 may be capable of undergoing a change in the volume of the red blood cell treatment agent container 53. In this case, for example, the red blood cell treatment agent container 53 includes a syringe that holds a fluid and a plunger which is inserted into the syringe and movable in the syringe, and the volume within the syringe that can hold the fluid can be changed by moving the plunger. Alternatively, the red blood cell treatment agent container 53 may be a flexible bellows or bag.

For example, the cell culture device according to the embodiment further includes a mixer 57 in which the blood, and the red blood cell precipitating agent or the red blood cell removal agent are mixed. For example, the mixer 57 includes a bent flow path through which a mixed solution containing the blood and the red blood cell precipitating agent or the red blood cell removal agent flows . The bent flow path may be bent in a spiral shape. The flow path may meander in the bent flow path. The cross-sectional area may repeatedly increase and decrease in the bent flow path. The mixer 57 may have a structure in which the interior can be closed from the outside air. The closed space including the interior of the mixer 57 may be configured such that gases, viruses, microorganisms, impurities and the like are not exchanged with the outside. The mixer 57 may be embedded and enclosed in a non-gas-permeable substance. At least a part of the mixer 57 may be formed by inscribing in a member. At least a part of the mixer 57 may be formed by overlaying recesses inscribed in members.

A flow path 51 for sending at least the blood from the blood container 50 to the mixer 57 is connected to the blood container 50. A valve other than the fluid machine may not be provided at the flow path 51. A flow path 54 for sending at least the red blood cell precipitating agent or the red blood cell removal agent from the red blood cell treatment agent container 53 to the mixer 57 is connected to the red blood cell treatment agent container 53. A valve other than the fluid machine may not be provided at the flow path 54. The flow path 51 and the flow path 54 merge with a flow path 56. A valve other than the fluid machine may not be provided at the flow path 56. The flow path 56 is connected to the mixer 57. A flow path 58 for sending the mixed solution containing the blood and the red blood cell precipitating agent or the red blood cell removal agent mixed in the mixer 57 into a red blood cell remover 11 is connected to the mixer 57. A valve other than the fluid machine may not be provided at the flow path 58.

A fluid machine 52 such as a pump for moving the fluid in the flow path 51 may be provided at the flow path 51. A positive displacement pump can be used as the fluid machine 52. Examples of positive displacement pumps include reciprocating pumps including a piston pump, a plunger pump, and a diaphragm pump, or rotary pumps including a gear pump, a vane pump, and a screw pump. Examples of diaphragm pumps include a turbing pump and a piezoelectric (piezo) pump. The turbing pump may be called a peristaltic pump. In addition, a microfluidic chip module in which various types of pumps are combined may be used. The same applies to other fluid machines in the present disclosure. In the case where a sealable type pump such as a peristaltic pump, a turbing pump, or a diaphragm pump is used, it is possible to send the fluid without the pump being in direct contact with the fluid inside the flow path. A fluid machine 55 such as a pump for moving the fluid in the flow path 54 may be provided at the flow path 54.

The flow paths 51, 54, 56, and 58 may have a structure in which the interior can be closed from the outside air. The closed space including the interior of the flow paths 51, 54, 56, and 58 may be configured such that gases, viruses, microorganisms, impurities and the like are not exchanged with the outside. The flow paths 51, 54, 56, and 58 may be embedded and enclosed in a non-gas-permeable substance. At least a part of the flow paths 51, 54, 56, and 58 may be formed by inscribing in a member. At least a part of the flow paths 51, 54, 56, and 58 may be formed by overlaying recesses inscribed in members.

In the case where the mixed solution containing the blood and the red blood cell precipitating agent or the red blood cell removal agent is sent to the red blood cell remover 11, the fluid machine 52 moves the blood in the blood container 50 into the mixer 57 via the flow paths 51 and 56. In addition, the fluid machine

55 moves the red blood cell precipitating agent or the red blood cell removal agent in the red blood cell treatment agent container 53 into the mixer 57 via the flow paths 54 and 56. Here, a fluid machine may not be provided at the flow paths 51 and 54, but a fluid machine may be provided at the flow path 56, and the fluid machine provided at the flow path 56 may move the blood in the blood container 50 and the red blood cell precipitating agent or the red blood cell removal agent in the red blood cell treatment agent container 53 into the mixer 57. In the mixer 57, the blood, and the red blood cell precipitating agent or the red blood cell removal agent are mixed. The mixed solution containing the blood and the red blood cell precipitating agent or the red blood cell removal agent mixed in the mixer 57 is sent to the red blood cell remover 11 via the flow path 58.

The red blood cell remover 11 may have a structure in which the interior can be closed from the outside air. The closed space including the interior of the red blood cell remover 11 may be configured such that gases, viruses, microorganisms, impurities and the like are not exchanged with the outside. The red blood cell remover 11 may be embedded and enclosed in a non-gas-permeable substance. At least a part of the red blood cell remover 11 may be formed by inscribing in a member. At least a part of the red blood cell remover 11 may be formed by overlaying recesses inscribed in members. In the red blood cell remover 11, the volume of the red blood cell remover 11 can be changed.

In the case where the blood is mixed with the red blood cell precipitating agent, red blood cells precipitate in the red blood cell remover 11, and the red blood cells are at least partially removed from the blood. In the case where the blood is mixed with the red blood cell removal agent, red blood cells in the red blood cell remover 11 may be hemolyzed, and red blood cells may be at least partially removed from the blood.

The cell culture device according to the embodiment may further include a mononuclear cell collector 15 which receives treated blood from which the red blood cells are at least partially removed from the red blood cell remover 11 and collects mononuclear cells from the treated blood. The mononuclear cell collector 15 may have a structure in which the interior can be closed from the outside air. The closed space including the interior of the mononuclear cell collector 15 may be configured such that gases, viruses, microorganisms, impurities and the like are not exchanged with the outside. The mononuclear cell collector 15 may be embedded and enclosed in a non-gas-permeable substance. At least a part of the mononuclear cell collector 15 may be formed by inscribing in a member. At least a part of the mononuclear cell collector 15 may be formed by overlaying recesses inscribed in members. In the mononuclear cell collector 15, the volume of the mononuclear cell collector 15 can be changed.

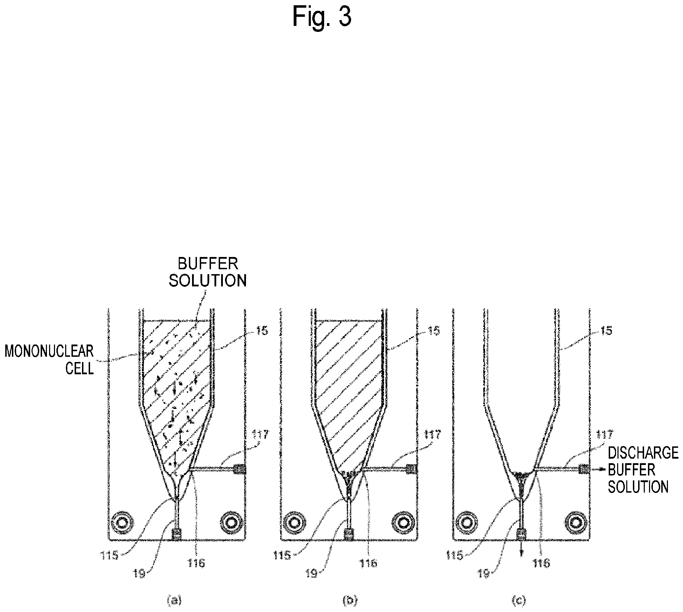

As shown in Fig. 3, for example, a first opening 115 is provided at the bottom of the mononuclear cell collector 15, and a second opening 116 is provided at the side surface of the mononuclear cell collector 15. The position of the first opening 115 may be below the second opening 116 in the direction of gravity.

A flow path 19 is connected to the first opening 115 of the mononuclear cell collector 15. A valve other than the fluid machine may not be provided at the flow path 19. The flow path 19 may have a structure in which the interior can be closed from the outside air. The closed space including the interior of the flow path 19 may be configured such that gases, viruses, microorganisms, impurities and the like are not exchanged with the outside. The flow path 19 may be embedded and enclosed in a non-gas-permeable substance. At least a part of the flow path 19 may be formed by inscribing in a member. At least a part of the flow path 19 may be formed by overlaying recesses inscribed in members.

A flow path 117 is connected to the second opening 116 of the mononuclear cell collector 15. A valve other than the fluid machine may not be provided at the flow path 117. The flow path 117 may have a structure in which the interior can be closed from the outside air. The closed space including the interior of the flow path 117 may be configured such that gases, viruses, microorganisms, impurities and the like are not exchanged with the outside. The flow path 117 may be embedded and enclosed in a non-gas-permeable substance. At least a part of the flow path 117 may be formed by inscribing in a member. At least a part of the flow path 117 may be formed by overlaying recesses inscribed in members. As shown in Fig. 1 and Fig. 2, a fluid machine 21 such as a pump for moving the fluid in the flow path 117 is provided at the flow path 117.

As shown in Fig. 3, the mononuclear cell collector 15 may have a funnel-shaped bottom. In this case, for example, the first opening 115 is provided at the tip of the funnel-shaped bottom of the mononuclear cell collector 15, and the second opening 116 is provided at the side surface of the funnel-shaped bottom. A filter through which mononuclear cells cannot pass may be provided at the second opening 116.

The interior of the mononuclear cell collector 15 can hold a diluting solution such as a buffer solution. As shown in Fig. 1 and Fig. 2, the diluting solution may be introduced into the mononuclear cell collector 15 via a flow path 60 from a diluting solution container 61 that holds the diluting solution. A valve other than the fluid machine may not be provided at the flow path 60. A fluid machine 62 such as a pump for moving the fluid in the flow path 60 may be provided at the flow path 60. The diluting solution container 61 may be capable of undergoing a change in the volume of the diluting solution container. In addition, for example, the interiors of the flow path 19 and the flow path 117 are filled with a diluting solution.

At least one of the diluting solution container 61 and the flow path 60 may have a structure in which the interior can be closed from the outside air. The closed space including the interior of the diluting solution container 61 and the flow path 60 may be configured such that gases, viruses, microorganisms, impurities and the like are not exchanged with the outside. The diluting solution container 61 and the flow path 60 may be embedded and enclosed in a non-gas-permeable substance. At least a part of the diluting solution container 61 and the flow path 60 may be formed by inscribing in a member. At least a part of the diluting solution container 61 and the flow path 60 may be formed by overlaying recesses inscribed in members.

A flow path 17 for sending the treated blood from which the red blood cells are at least partially removed from the red blood cell remover 11 to the mononuclear cell collector 15 is provided between the red blood cell remover 11 and the mononuclear cell collector 15. A valve other than the fluid machine may not be provided at the flow path 17. The flow path 17 may have a structure in which the interior can be closed from the outside air. The closed space including the interior of the flow path 17 may be configured such that gases, viruses, microorganisms, impurities and the like are not exchanged with the outside. The flow path 17 may be embedded and enclosed in a non-gas-permeable substance. At least a part of the flow path 17 may be formed by inscribing in a member. At least a part of the flow path 17 may be formed by overlaying recesses inscribed in members.

A fluid machine 18 such as a pump for moving the fluid in the flow path 17 is provided at the flow path 17.

In the case where a gas and a diluting solution are filled into the mononuclear cell collector 15 in advance, the fluid machine 18 aspirates the treated blood from which red blood cells are at least partially removed into the red blood cell remover 11 via the flow path 17 and supplies the aspired treated blood from which red blood cells are at least partially removed into the mononuclear cell collector 15.

In the case where the red blood cells are precipitated in the red blood cell remover 11, the supernatant in the red blood cell remover 11 is sent to the mononuclear cell collector 15 as the treated blood from which the red blood cells are at least partially removed.

The treated blood from which the red blood cells are at least partially removed, which has been sent to the mononuclear cell collector 15, is diluted with the diluting solution as shown in Fig. 3(a). In the diluted treated blood solution, platelets float, and mononuclear cells precipitate toward the bottom of the mononuclear cell collector 15. Here, the diluting solution may contain a red blood cell removal agent. In this case, red blood cells remaining in the treated blood solution are hemolyzed. Alternatively, a red blood cell treatment agent container different from the red blood cell treatment agent container 53 is connected to the mononuclear cell collector 15 via a flow path, and a red blood cell precipitating agent or a red blood cell removal agent may be supplied to the mononuclear cell collector 15 from the red blood cell treatment agent container.

As shown in Fig. 3(b), the precipitated mononuclear cells accumulate at the tip of the funnel-shaped bottom of the mononuclear cell collector 15. After mononuclear cells precipitate in the diluted treated blood solution, as shown in Fig. 3(c), the fluid machine 21 provided at the flow path 117 connected to the second opening 116 of the mononuclear cell collector 15 aspirates the diluted treated blood solution which is the supernatant. The aspiration power for aspirating the supernatant is set so that it is difficult to aspirate the precipitated mononuclear cells. The supernatant contains the platelets and the hemolyzed red blood cells. Therefore, in the case where the supernatant is removed from the mononuclear cell collector 15 by aspiration, it is possible to separate the mononuclear cells from the platelets and the red blood cells. The aspirated supernatant may be sent into the red blood cell remover 11 via a flow path 216 connected to the fluid machine 21 shown in Fig. 1 and Fig. 2. A valve other than the fluid machine may not be provided at the flow path 216. Alternatively, the aspirated supernatant may be sent to a second variable volume container 30 to be described below or may be sent to another container. Then, supply of the diluting solution from the diluting solution container 61 to the mononuclear cell collector 15 and aspiration of the supernatant may be repeatedly performed. The excess fluid in the red blood cell remover 11 may be sent to the second variable volume container 30 to be described below via a flow path 93. A valve other than the fluid machine may not be provided at the flow path 93.

A mononuclear cell aspiration device 20 that aspirates the mononuclear cells accumulated at the bottom of the mononuclear cell collector 15 is provided at the flow path 19. A fluid machine such as a pump can be used as the mononuclear cell aspiration device 20. For example, the size of the first opening 115 shown in Fig. 3 is set so that, when the mononuclear cell aspiration device 20 does not aspirate mononuclear cells, the mononuclear cells are clogged in the first opening 115, and when the mononuclear cell aspiration device 20 aspirates mononuclear cells, the mononuclear cells can pass through the first opening 115. In the case where the mononuclear cell aspiration device 20 aspirates mononuclear cells, the mononuclear cells move from the interior of the mononuclear cell collector 15 to the flow path 19.

Here, by pressurizing the interior of the mononuclear cell collector 15, the mononuclear cells in the mononuclear cell collector 15 may be moved to the flow path 19. In this case, the mononuclear cell aspiration device 20 may or may not be provided at the flow path 19.

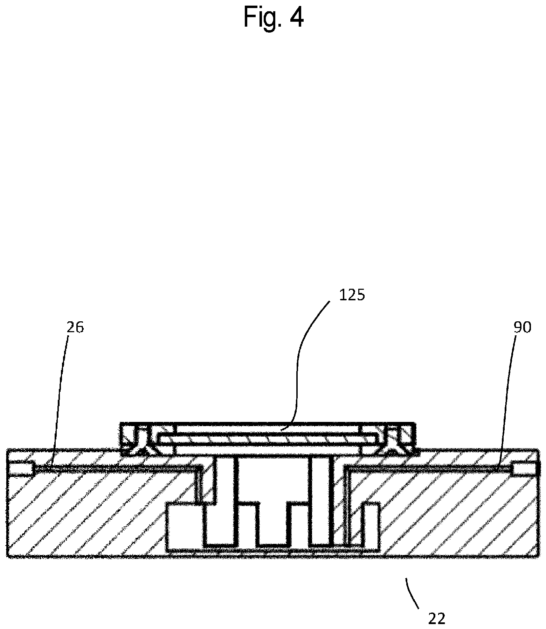

The cell culture vessel 22 for culturing cells shown in Fig. 1 and Fig. 2 may have a structure in which the interior can be closed from the outside air as shown in Fig. 4. The closed space including the interior of the cell culture vessel 22 may be configured such that gases, viruses, microorganisms, impurities and the like are not exchanged with the outside. The cell culture vessel 22 may be embedded and enclosed in a non-gas-permeable substance. At least a part of the cell culture vessel 22 may be formed by inscribing in a member. At least a part of the cell culture vessel 22 may be formed by overlaying recesses inscribed in members .

In the cell culture vessel 22, cells may be adhesive-cultured, or cells may be suspended-cultured. In the case where cells are adhesive-cultured, the interior of the cell culture vessel 22 may be coated with a cell adhesion coating agent such as matrigel, collagen, polylysine, fibronectin, vitronectin, gelatin, or laminin. Adhesive-culture will be described below as an example. The interior of the cell culture vessel 22 may be separated by a medium component permeable member through which cells cannot permeate but medium components and waste products can permeate. The side wall of the cell culture vessel 22 may be coated with a non-cell-adhesive substance such as poly(2-hydroxyethyl methacrylate) (poly-HEMA) so that cells do not adhere, and the side wall of the cell culture vessel 22 may be non-adhesive to cells. A transparent window through which the interior can be observed may be provided in the cell culture vessel 22. As the material of the window, for example, glass or a resin can be used. For example, a transparent window 125 is provided on at least one of the bottom surface and the top surface of the cell culture vessel 22. Therefore, the interior can be observed from the bottom surface side of the cell culture vessel 22 using a microscope or the like.

A temperature adjuster for heating and cooling a window may be provided in the cell culture vessel 22. The temperature adjuster may be a transparent heater such as a transparent conductive film that is disposed on the window and heats the window. Alternatively, the cell culture vessel 22 may include a temperature adjuster for heating and cooling a housing. In the case where the temperature of the housing is adjusted by the temperature adjuster, it is possible to adjust the temperature of a medium in the cell culture vessel 22. The cell culture vessel 22 may further include a thermometer that measures the temperature of the medium in the cell culture vessel 22. The thermometer may measure the temperature of the medium based on the temperature of the cell culture vessel 22 without contacting with the medium, or may be in contact with the medium and directly measure the temperature of the medium. In this case, the temperature adjuster may be feedback-controlled so that the temperature of the medium becomes a predetermined temperature. The temperature of the medium is adjusted, for example, from 0°C to 45°C, or from 20°C to 45°C.

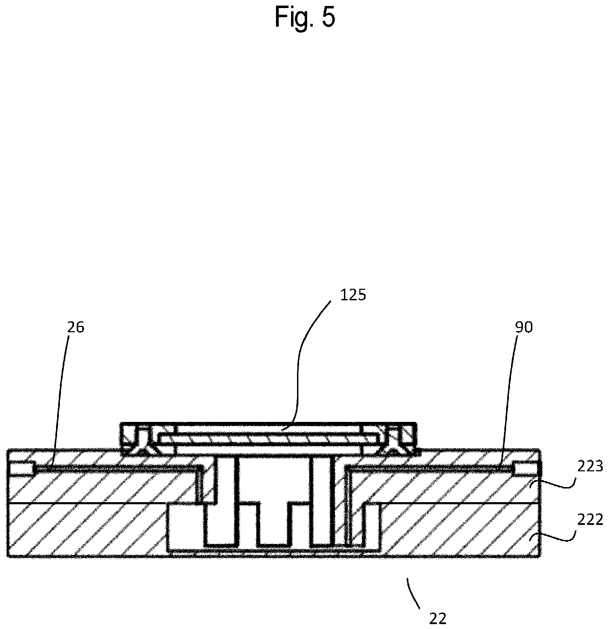

The cell culture vessel 22 may be integrally molded. The cell culture vessel 22 may be produced by a 3D printer method. Examples of 3D printer methods include a material extrusion deposition method, a material jetting method, a binder jetting method and an optically shaping method. Alternatively, as shown in Fig. 5, the cell culture vessel 22 includes a first housing 222 having a bottom surface and a second housing 223 which is disposed on the first housing 222 and has a top surface that faces the bottom surface, and the first housing 222 and the second housing 223 may be combined to form the interior. The flow path connected to the cell culture vessel 22 may be provided in at least one of the first housing 222 and the second housing 223. A petri dish or the like may be disposed as an internal culture container interior the cell culture vessel 22. In this case, the flow path is configured to supply a fluid into the internal culture container.

As shown in Fig. 1 and Fig. 2, the flowpath 19 is connected to the cell culture vessel 22. Cells are sent into the cell culture vessel 22 via the flow path 19. For example, the flow path 19 is connected to the side wall of the cell culture vessel 22. A flow path 23 is connected to the flow path 19. A valve other than the fluid machine may not be provided at the flow path 23. The flow path 23 may have a structure in which the interior can be closed from the outside air. The closed space including the interior of the flow path 23 may be configured such that gases, viruses, microorganisms, impurities and the like are not exchanged with the outside. The flow path 23 may be embedded and enclosed in a non-gas-permeable substance. At least a part of the flow path 23 may be formed by inscribing in a member. At least a part of the flow path 23 may be formed by overlaying recesses inscribed in members. A fluid machine 24 such as a pump for moving the fluid in the flow path 23 is provided at the flow path 23.

For example, a first medium container 25 which is a fluid container that holds a somatic cell medium such as a differentiated cell medium or a stem cell medium suitable for iPS cells, ES cells, stem cells and the like is connected to the flow path 23. The medium may be a gel, a liquid, or a fluid solid. Examples of fluid solids include agar and a temperature-sensitive gel.

In the case where the medium is gel form, the medium may contain a polymer compound. For example, the polymer compound may be at least one selected from the group consisting of gellan gum, deacylated gellan gum, hyaluronic acid, ramsan gum, diutan gum, xanthan gum, carrageenan, fucoidan, pectin, pectic acid, pectinic acid, heparan sulfate, heparin, heparitin sulfate, keratosulfate, chondroitin sulfate, dermatan sulfate, rhamnan sulfate, and salts thereof. In addition, the medium may contain methyl cellulose. In the case where the medium contains methyl cellulose, aggregation between cells is further reduced.

Alternatively, the medium may contain a small amount of a temperature-sensitive gel selected from among poly(glycerol monomethacrylate) (PGMA), poly(2-hydroxypropyl methacrylate) (PHPMA), poly(N-isopropylacrylamide) (PNIPAM), amine terminated, carboxylic acid terminated, maleimide terminated, N-hydroxysuccinimide (NHS) ester terminated, triethoxysilane terminated, poly(N-isopropylacrylamide-co-acrylamide), poly(N-isopropy lacrylamide-co-acrylic acid), poly(N-isopropylacrylamide-co-butylacrylate), poly(N-isopropylacrylamide-co-methacrylic acid), poly(N-isopropylacrylamide-co-methacrylic acid-co-octadecyl acrylate), and N-Isopropylacrylamide.

Here, in the present disclosure, the gel form medium or gel medium includes a polymer medium.

In the case where the cells that are sent from the flow path 19 into the cell culture vessel 22 are mononuclear cells which are somatic cells, for example, a blood cell medium can be used as the somatic cell medium. The first medium container 25 may have a structure in which the interior can be closed from the outside air. The closed space including the interior of the first medium container 25 may be configured such that gases, viruses, microorganisms, impurities and the like are not exchanged with the outside. The first medium container 25 may be embedded and enclosed in a non-gas-permeable substance. At least a part of the first medium container 25 may be formed by inscribing in a member. At least a part of the first medium container 25 may be formed by overlaying recesses inscribed in members. The first medium container 25 may be capable of undergoing a change in the volume of the first medium container 25. In this case, for example, the first medium container 25 includes a syringe that holds a somatic cell medium and a plunger which is inserted into the syringe and movable in the syringe, and the volume within the syringe that can hold the somatic cell medium can be changed by moving the plunger. Alternatively, the first medium container 25 may be a flexible bellows or bag.

In the case where the mononuclear cells are sent from the mononuclear cell collector 15 to the flow path 19, the fluid machine 24 sends the somatic cell medium from the first medium container 25 to the flow path 19 through the flow path 23. The first medium container 25 may reduce the volume that can hold the somatic cell medium. Here, the first medium container 25 may actively contract its volume or passively contract its volume by suction force from the interior of the flow path 23. The somatic cell medium sent to the flow path 19 via the flow path 23 is mixed with the mononuclear cells in the flow path 19 and sent into the cell culture vessel 22. Here, the mononuclear cells prepared in advance may be supplied into the cell culture vessel 22. In addition, the cells sent to the cell culture vessel 22 are not limited to mononuclear cells, and may be any cells such as somatic cells.

A temperature adjusting device configured to adjust the temperature of the medium in the first medium container 25 may be provided at at least one of the first medium container 25 and the flow path 23. Even after the cells are sent into the cell culture vessel 22, the fluid machine 24 may send the somatic cell medium from the first medium container 25 into the cell culture vessel 22.

For example, the first variable volume container 27 is connected to the cell culture vessel 22 via the flow path 26. The flow path 26 may have a structure in which the interior can be closed from the outside air. The closed space including the interior of the flow path 26 may be configured such that gases, viruses, microorganisms, impurities and the like are not exchanged with the outside. The flow path 26 may be embedded and enclosed in a non-gas-permeable substance. At least a part of the flow path 26 may be formed by inscribing in a member. At least a part of the flow path 26 may be formed by overlaying recesses inscribed in members. A fluid machine 28 such as a pump for moving the fluid in the flow path 26 may be provided at the flow path 26.

The first variable volume container 27 may have a structure in which the interior can be closed from the outside air. The closed space including the interior of the first variable volume container 27 may be configured such that gases, viruses, microorganisms, impurities and the like are not exchanged with the outside. The first variable volume container 27 may be embedded and enclosed in a non-gas-permeable substance. At least a part of the first variable volume container 27 may be formed by inscribing in a member. At least a part of the first variable volume container 27 may be formed by overlaying recesses inscribed in members. The first variable volume container 27 may be capable of undergoing a change in the volume of the first variable volume container 27. In this case, for example, the first variable volume container 27 includes a syringe that holds a fluid and a plunger which is inserted into the syringe and movable in the syringe, and the volume within the syringe that can hold the fluid can be changed by moving the plunger. Alternatively, the first variable volume container 27 may be a flexible bellows or bag.

For example, the second variable volume container 30 is connected to the cell culture vessel 22 via a flow path 29. For example, the flow path 29 is connected to the side wall of the cell culture vessel 22. The flow path 29 may have a structure in which the interior can be closed from the outside air. The closed space including the interior of the flow path 29 may be configured such that gases, viruses, microorganisms, impurities and the like are not exchanged with the outside. The flow path 29 may be embedded and enclosed in a non-gas-permeable substance. At least a part of the flow path 29 may be formed by inscribing in a member. At least a part of the flow path 29 may be formed by overlaying recesses inscribed in members. A fluid machine such as a pump for moving the fluid in the flow path 29 may be provided at the flow path 29. A valve other than the fluid machine may not be provided at the flow path 29.

The second variable volume container 30 may have a structure in which the interior can be closed from the outside air. The closed space including the interior of the second variable volume container 30 may be configured such that gases, viruses, microorganisms, impurities and the like are not exchanged with the outside. The second variable volume container 30 may be embedded and enclosed in a non-gas-permeable substance. At least a part of the second variable volume container 30 may be formed by inscribing in a member. At least a part of the second variable volume container 30 may be formed by overlaying recesses inscribed in members. In the second variable volume container 30, the volume of the second variable volume container 30 can be changed. In this case, for example, the second variable volume container 30 includes a syringe that holds a fluid and a plunger which is inserted into the syringe and movable in the syringe, and the volume within the syringe that can hold the fluid can be changed by moving the plunger. Alternatively, the second variable volume container 30 may be a flexible bellows or bag.

In the case where the somatic cells and the somatic cell medium are sent into the cell culture vessel 22 from the flow path 19, a gas such as air in the cell culture vessel 22 moves, for example, in the second variable volume container 30, and the second variable volume container 30 expands its volume, and receives the gas that has moved from the interior of the cell culture vessel 22. Here, the second variable volume container 30 may actively expand or passively expand its volume upon receiving pressure.

The first variable volume container 27, for example, holds a substance such as a factor that induces a cell in a first state into a cell in a second state such as an inducing factor in the interior. The inducing factor may be RNA, a protein, or a compound. RNA may be modified RNA or unmodified RNA. The first variable volume container 27 may accommodate, for example, a lipofectamine reagent. The inducing factor may be contained in a plasmid vector or a virus vector such as a retrovirus vector, a lentivirus vector, or a Sendai virus vector or in viruses, or in a mixture thereof. In the present disclosure, induction refers to reprogramming, initialization, transformation, transdifferentiation (Transdifferentiation or Lineage reprogramming), differentiation induction, cell fate change (Cell fate reprogramming), or the like. The reprogramming factor includes, for example, OCT3/4, SOX2, KLF4, and c-MYC.

In the case where an inducing factor such as a reprogramming factor is introduced into the somatic cells to prepare iPS cells, the fluid machine 28 moves the somatic cell medium containing the somatic cells in the cell culture vessel 22 into the first variable volume container 27 via the flow path 26. In addition, the first variable volume container 27 expands its volume and receives the somatic cell medium containing the somatic cells. Here, the first variable volume container 27 may actively expand its volume or passively expand its volume upon receiving pressure. The volume of the second variable volume container 30 holding a gas is contracted, and the accommodated gas is sent into the cell culture vessel 22. Here, the second variable volume container 30 may actively contract its volume or may passively contract its volume by suction force from the interior of the cell culture vessel 22.

In the case where the somatic cells move from the interior of the cell culture vessel 22 into the first variable volume container 27, they come into contact with the inducing factor in the first variable volume container 27, and the inducing factor is introduced into the somatic cells. Here, the first variable volume container 27 may repeatedly expand and contract its volume and stir the somatic cell medium containing the somatic cells and the inducing factor.

For example, a coating agent container 82 which is a fluid container that holds, for example, a cell adhesion coating agent such as matrigel, collagen, polylysine, fibronectin, vitronectin, gelatin, or laminin is connected to the cell culture vessel 22 via a flow path 81. For example, the flow path 81 is connected to the side wall of the cell culture vessel 22.

The flow path 81 may have a structure in which the interior can be closed from the outside air. The closed space including the interior of the flow path 81 may be configured such that gases, viruses, microorganisms, impurities and the like are not exchanged with the outside. The flow path 81 may be embedded and enclosed in a non-gas-permeable substance. At least a part of the flow path 81 may be formed by inscribing in a member. At least a part of the flow path 81 may be formed by overlaying recesses inscribed in members. A fluid machine 83 such as a pump for moving the fluid in the flow path 81 may be provided at the flow path 81. A valve other than the fluid machine may not be provided at the flow path 81.

The coating agent container 82 may have a structure in which the interior can be closed from the outside air. The closed space including the interior of the coating agent container 82 may be configured such that gases, viruses, microorganisms, impurities and the like are not exchanged with the outside. The coating agent container 82 may be embedded and enclosed in a non-gas-permeable substance. At least a part of the coating agent container 82 may be formed by inscribing in a member. At least a part of the coating agent container 82 may be formed by overlaying recesses inscribed in members. In the coating agent container 82, the volume of the coating agent container 82 can be changed. In this case, for example, the coating agent container 82 includes a syringe that holds a fluid and a plunger which is inserted into the syringe and movable in the syringe, and the volume within the syringe that can hold the fluid can be changed by moving the plunger. Alternatively, the coating agent container 82 may be a flexible bellows or bag.

During the cells are introduced with the factor in the first variable volume container 27, the fluid machine 83 moves the cell adhesion coating agent in the coating agent container 82 into the cell culture vessel 22 via the flow path 81. Thereby, the bottom surface of the cell culture vessel 22 is covered with the cell adhesion coating agent.

In the case where the cell adhesion coating agent is sent from the flow path 81 into the cell culture vessel 22, the excess fluid in the cell culture vessel 22 moves, for example, in the second variable volume container 30, and the second variable volume container 30 expands its volume and receives the fluid that has moved from the interior of the cell culture vessel 22. Here, the second variable volume container 30 may actively expand or passively expand its volume upon receiving pressure.

A flow path 129 may be provided between the cell culture vessel 22 and the second variable volume container 30. For example, the flow path 129 is connected to the side wall of the cell culture vessel 22. The flow path 129 may have a structure in which the interior can be closed from the outside air. The closed space including the interior of the flow path 129 may be configured such that gases, viruses, microorganisms, impurities and the like are not exchanged with the outside. The flow path 129 may be embedded and enclosed in a non-gas-permeable substance. At least a part of the flow path 129 may be formed by inscribing in a member. At least a part of the flow path 129 may be formed by overlaying recesses inscribed in members . A fluid machine 130 such as a pump for moving the fluid in the flow path 129 may be provided at the flow path 129. A valve other than the fluid machine may not be provided at the flow path 129.

After the bottom surface of the cell culture vessel 22 is covered with the cell adhesion coating agent for a predetermined time, the fluid machine 130 moves the cell adhesion coating agent in the cell culture vessel 22 into the second variable volume container 30 via the flow path 129.

After the cell adhesion coating agent moves into the second variable volume container 30, the fluid machine 28 moves the somatic cell medium containing the somatic cells into which the inducing factor in the first variable volume container 27 is introduced into the cell culture vessel 22 via the flow path 26. The first variable volume container 27 contracts its volume. In addition, the second variable volume container 30 expands its volume and receives a gas and/or liquid from the interior of the cell culture vessel 22.

In the case where the cell adhesion coating agent is applied to the bottom surface of the cell culture vessel 22 in advance or the cell culture vessel 22 contains the cell adhesion coating agent in advance, the coating agent container 82 may not be provided.

As another example, without moving the cells in the cell culture vessel 22 into the first variable volume container 27, the fluid machine 28 may move the inducing factor in the first variable volume container 27 into the cell culture vessel 22 containing the cells via the flow path 26. In this case, the first variable volume container 27 may contract its volume, and the second variable volume container 30 may expand its volume. In the case where the inducing factor moves from the interior of the first variable volume container 27 into the cell culture vessel 22, it comes into contact with the somatic cells in the cell culture vessel 22 and the inducing factor is introduced into the somatic cells. Here, the fluid machine 28 may move the inducing factor in the first variable volume container 27 into the cell culture vessel 22 via the flow path 26 separately a plurality of times. Thereby, the inducing factor is introduced into the somatic cells separately a plurality of times.

For example, a second medium container 32 which is a fluid container that holds, for example, a medium such as a stem cell medium or a somatic cell medium is connected to the cell culture vessel 22 via a flow path 31. For example, the flow path 31 is connected to the side wall of the cell culture vessel 22. In the following, an example in which a stem cell medium is held in the second medium container 32 will be described. The stem cell medium may be a gel, a liquid, or a fluid solid. The stem cell medium may contain agar and a temperature-sensitive gel. An induction culture medium, an expansion culture medium, or a maintenance culture medium can be used as the stem cell medium.

The flow path 31 may have a structure in which the interior can be closed from the outside air. The closed space including the interior of the flow path 31 may be configured such that gases, viruses, microorganisms, impurities and the like are not exchanged with the outside. The flow path 31 may be embedded and enclosed in a non-gas-permeable substance. At least a part of the flow path 31 may be formed by inscribing in a member. At least a part of the flow path 31 may be formed by overlaying recesses inscribed in members. A fluid machine 33 such as a pump for moving the fluid in the flow path 31 may be provided at the flow path 31. A valve other than the fluid machine may not be provided at the flow path 31.

The second medium container 32 may have a structure in which the interior can be closed from the outside air. The closed space including the interior of the second medium container 32 may be configured such that gases, viruses, microorganisms, impurities and the like are not exchanged with the outside. The second medium container 32 may be embedded and enclosed in a non-gas-permeable substance. At least a part of the second medium container 32 may be formed by inscribing in a member. At least a part of the second medium container 32 may be formed by overlaying recesses inscribed in members. In the second medium container 32, the volume of the second medium container 32 can be changed. In this case, for example, the second medium container 32 includes a syringe that holds a fluid and a plunger which is inserted into the syringe and movable in the syringe, and the volume within the syringe that can hold the fluid can be changed by moving the plunger. Alternatively, the second medium container 32 may be a flexible bellows or bag.

A temperature adjusting device configured to adjust the temperature of the medium in the second medium container 32 may be provided at at least one of the second medium container 32 and the flow path 31.

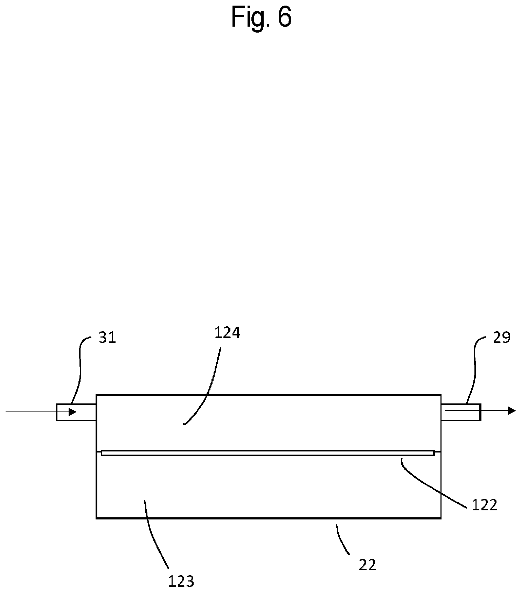

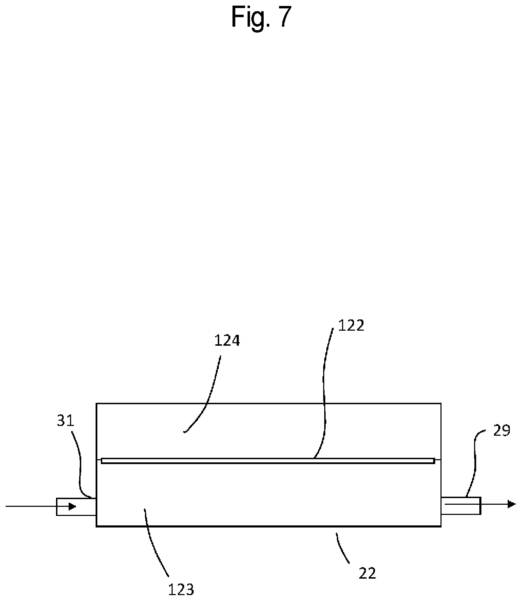

After a predetermined time has elapsed since the inducing factor is introduced into the somatic cells, the fluid machine 33 moves the stem cell medium in the second medium container 32 into the cell culture vessel 22 via the flow path 31. As shown in Fig. 6, the stem cell medium may be put into a section 124 which is in contact with a section 123 in which the cells exist and in which cells do not exist on the upper side in the direction of gravity among sections separated by a medium component permeable member 122 in the cell culture vessel 22. Alternatively, as shown in Fig. 7, the stem cell medium may be put into the section 123 in which cells do not exit on the lower side in the direction of gravity among sections separated by the medium component permeable member 122 in the cell culture vessel 22. In this case, the cells exist in the section 124 on the upper side in the direction of gravity. The second medium container 32 shown in Fig. 1 and Fig. 2 with which the stem cell medium is aspirated from the interior contracts its volume. Here, the second medium container 32 may actively contract its volume or passively contract its volume.

In the case where the stem cell medium is sent from the flow path 31 into the cell culture vessel 22, a gas such as air and the medium in the cell culture vessel 22 moves into the second variable volume container 30 via, for example, the flow path 29, and the second variable volume container 30 expands its volume and receives the gas and the medium that have moved from the interior of the cell culture vessel 22. Here, the second variable volume container 30 may actively expand its volume or passively expand its volume upon receiving pressure.

The flow path 29 may be in connected to a section in which cells do not exist and contacting with a section in which the cells exist among sections separated by the medium component permeable member in the cell culture vessel 22. Alternatively, the flow path 29 may be in contact with a section in which the cells exist among sections separated by the medium component permeable member in the cell culture vessel 22. In this case, excess cells in the cell culture vessel 22 may be sent to the second variable volume container 30 via the flow path 29.

Among sections separated by the medium component permeable member in the cell culture vessel 22, the medium in the section in which the cells exist and the medium in the section in which cells do not exist exchange medium components and waste products due to, for example, osmotic pressure. For example, a semipermeable membrane, a mesh, or a hollow fiber membrane can be used as the medium component permeable member. The semipermeable membrane includes a dialysis membrane. The medium component permeable member may be fixed into the cell culture vessel 22 via a packing or the like. Among sections separated by the medium component permeable member in the cell culture vessel 22, at least one of the flow paths 19, 26, 90, and 129 may communicate with the section in which cells exist, and at least one of the flow paths 29, 31, 81, 84, and 87 may communicate with the section in which cells do not exit. Alternatively, one or more hollow fibers may be disposed in the cell culture vessel 22 and cells may be disposed interior the hollow fiber. In this case, for example, at least one of the flow paths 19, 26, 90, and 129 may communicate with the interior of the hollow fiber, and at least one of the flow paths 29, 31, 81, 84, and 87 may communicate with the outside of the hollow fiber.

In the case where the medium component permeable member is a semipermeable membrane, the molecular weight cutoff of the semipermeable membrane is, for example, 0.1 KDa or more, 10 KDa or more, or 50 KDa or more. Examples of semipermeable membranes include cellulose ester, ethyl cellulose, cellulose esters, regenerated cellulose, polysulfone, polyacrylonitrile, polymethylmethacrylate, ethylene vinyl alcohol copolymers, polyester polymer alloys, polycarbonate, polyamide, cellulose acetate, cellulose diacetate, cellulose triacetate, cuprammonium rayon, saponified cellulose, hemophan membranes, phosphatidylcholine membranes, and vitamin E coating films.

In the case where the medium component permeable member is a mesh, the mesh has smaller pores than the cells cultured in the cell culture vessel 22. The material of the mesh is, for example, a resin or a metal, but it is not particularly limited. The surface of the medium component permeable member may be non-cell-adhesive.

In the case where the medium component permeable member is a hollow fiber membrane, the hollow fiber membrane has smaller pores than the cells cultured in the cell culture vessel 22. For example, cells may be cultured interior the hollow fiber membrane.

During the cells are cultured in the cell culture vessel 22, the fluid machine 33 may move the stem cell medium in the second medium container 32 into the cell culture vessel 22 via the flow path 31 at a predetermined timing. In addition, the medium may be circulated between the second medium container 32 and the cell culture vessel 22. The second variable volume container 30 may expand its volume, and receive the excess stem cell medium used in the cell culture vessel 22 due to inflow of a new stem cell medium. The fluid machine 33 may control the amount of the medium sent based on, for example, the state of the medium, the state of the cell mass in the medium, the number of cells, the number of cell masses, the turbidity of the medium, and the change in pH, and may start and end transfer of the medium.

For example, a detachment solution container 85 which is a fluid container that holds the cell dissociation reagent such as trypsin, TrypLE Select, Accutase, and EDTA is connected to the cell culture vessel 22 via a flow path 84. For example, the flow path 84 is connected to the side wall of the cell culture vessel 22.

The flow path 84 may have a structure in which the interior can be closed from the outside air. The closed space including the interior of the flow path 84 may be configured such that gases, viruses, microorganisms, impurities and the like are not exchanged with the outside. The flow path 84 may be embedded and enclosed in a non-gas-permeable substance. At least a part of the flow path 84 may be formed by inscribing in a member. At least a part of the flow path 84 may be formed by overlaying recesses inscribed in members. A fluid machine 86 such as a pump for moving the fluid in the flow path 84 may be provided at the flow path 84. The flow path 84 is connected. A valve other than the fluid machine may not be provided at the flow path 84.

The detachment solution container 85 may have a structure in which the interior can be closed from the outside air. The closed space including the interior of the detachment solution container 85 may be configured such that gases, viruses, microorganisms, impurities and the like are not exchanged with the outside. The detachment solution container 85 may be embedded and enclosed in a non-gas-permeable substance. At least a part of the detachment solution container 85 may be formed by inscribing in a member. At least a part of the detachment solution container 85 may be formed by overlaying recesses inscribed in members. In the detachment solution container 85, the volume of the detachment solution container 85 can be changed. In this case, for example, the detachment solution container 85 includes a syringe that holds a fluid and a plunger which is inserted into the syringe and movable in the syringe, and the volume within the syringe that can hold the fluid can be changed by moving the plunger. Alternatively, the detachment solution container 85 may be a flexible bellows or bag.

A temperature adjusting device configured to adjust the temperature of the cell dissociation reagent in the detachment solution container 85 may be provided at at least one of the detachment solution container 85 and the flow path 84.

The fluid machine 86 moves the cell dissociation reagent in the detachment solution container 85 into the cell culture vessel 22 via the flow path 84. Thereby, the cells adhering to the bottom surface of the cell culture vessel 22 are exposed to the cell dissociation reagent.

In the case where the cell dissociation reagent is sent from the flow path 84 into the cell culture vessel 22, the excess fluid in the cell culture vessel 22 moves into, for example, the second variable volume container 30, and the second variable volume container 30 expands its volume and receives the fluid that has moved from the interior of the cell culture vessel 22. Here, the second variable volume container 30 may actively expand its volume or passively expand its volume upon receiving pressure.

After the cells in the cell culture vessel 22 are exposed to the cell dissociation reagent at a predetermined temperature for a predetermined time, the fluid machine 130 moves the cell dissociation reagent in the cell culture vessel 22 into the second variable volume container 30 via the flow path 129. In addition, after a predetermined time has elapsed at a predetermined temperature, the cells are detached from the bottom surface of the cell culture vessel 22. Some or all of the detached cells in the cell culture vessel 22 may be sent to the second variable volume container 30 via the flow path 129. At least some of the cells sent to the outside of the cell culture vessel 22 may be returned into the cell culture vessel 22. Then, the stem cell medium is supplied from the second medium container 32 into the cell culture vessel 22. After the cells adhere to the bottom surface of the cell culture vessel 22 and additionally a predetermined time has elapsed, for example, a Sendai virus vector may be eliminated at a high temperature such as 38°C. The introduction of the factor into the cells may be repeated a plurality of times, such as twice or three times. In this manner, the cell culture vessel and the cell culture device according to the present embodiment may function as a cell induction container and a cell induction device. Then, the cells in the cell culture vessel 22 are detached from the bottom surface of the cell culture vessel 22.

For example, a cryopreservation solution container 88 which is a fluid container that holds the cell cryopreservation solution is connected to the cell culture vessel 22 via a flow path 87. For example, the flow path 87 is connected to the side wall of the cell culture vessel 22.

The flow path 87 may have a structure in which the interior can be closed from the outside air. The closed space including the interior of the flow path 87 may be configured such that gases, viruses, microorganisms, impurities and the like are not exchanged with the outside. The flow path 87 may be embedded and enclosed in a non-gas-permeable substance. At least a part of the flow path 87 may be formed by inscribing in a member. At least a part of the flow path 87 may be formed by overlaying recesses inscribed in members. A fluid machine 89 such as a pump for moving the fluid in the flow path 87 may be provided at the flow path 87. A valve other than the fluid machine may not be provided at the flow path 87.

The cryopreservation solution container 88 may have a structure in which the interior can be closed from the outside air. The closed space including the interior of the cryopreservation solution container 88 may be configured such that gases, viruses, microorganisms, impurities and the like are not exchanged with the outside. The cryopreservation solution container 88 may be embedded and enclosed in a non-gas-permeable substance. At least a part of the cryopreservation solution container 88 may be formed by inscribing in a member. At least a part of the cryopreservation solution container 88 may be formed by overlaying recesses inscribed in members. In the cryopreservation solution container 88, the volume of the cryopreservation solution container 88 can be changed. In this case, for example, the cryopreservation solution container 88 includes a syringe that holds a fluid and a plunger which is inserted into the syringe and movable in the syringe, and the volume within the syringe that can hold the fluid can be changed by moving the plunger. Alternatively, the cryopreservation solution container 88 may be a flexible bellows or bag.

For example, after iPS cells are produced from the somatic cells into which the inducing factor was introduced in the cell culture vessel 22, the fluid machine 89 moves the cell cryopreservation solution in the cryopreservation solution container 88 into the cell culture vessel 22 via the flow path 87. Thereby, the cells in the cell culture vessel 22 are contained in the cell cryopreservation solution.

In the case where the cell cryopreservation solution is sent from the flow path 87 into the cell culture vessel 22, the excess fluid in the cell culture vessel 22 moves into, for example, the second variable volume container 30, and the second variable volume container 30 expands its volume and receives the fluid that has moved from the interior of the cell culture vessel 22. Here, the second variable volume container 30 may actively expand its volume or passively expand its volume upon receiving pressure.

For example, a cell cryopreservation container 91 which is a fluid container that holds a cell cryopreservation solution is connected to the cell culture vessel 22 via a flow path 90. For example, the flow path 90 is connected to the side wall of the cell culture vessel 22.

The flow path 90 may have a structure in which the interior can be closed from the outside air. The closed space including the interior of the flow path 90 may be configured such that gases, viruses, microorganisms, impurities and the like are not exchanged with the outside. The flow path 90 may be embedded and enclosed in a non-gas-permeable substance. At least a part of the flow path 90 may be formed by inscribing in a member. At least a part of the flow path 90 may be formed by overlaying recesses inscribed in members. A fluid machine 92 such as a pump for moving the fluid in the flow path 90 may be provided at the flow path 90. A valve other than the fluid machine may not be provided at the flow path 90.

The cell cryopreservation container 91 may have a structure in which the interior can be closed from the outside air. The closed space including the interior of the cell cryopreservation container 91 may be configured such that gases, viruses, microorganisms, impurities and the like are not exchanged with the outside. The cell cryopreservation container 91 may be embedded and enclosed in a non-gas-permeable substance. At least a part of the cell cryopreservation container 91 may be formed by inscribing in a member. At least a part of the cell cryopreservation container 91 may be formed by overlaying recesses inscribed in members. The cell cryopreservation container 91 may be capable of undergoing a change in the volume of the cell cryopreservation container 91. In this case, for example, the cell cryopreservation container 91 includes a syringe that holds a fluid and a plunger which is inserted into the syringe and movable in the syringe, and the volume within the syringe that can hold the fluid can be changed by moving the plunger. Alternatively, the cell cryopreservation container 91 may be a flexible bellows or bag.

The fluid machine 92 sends the cell cryopreservation solution containing cells in the cell culture vessel 22 to the cell cryopreservation container 91. The cell cryopreservation container 91 can be removed from the flow path 90 and sealed. The cell cryopreservation container 91 is disposed in, for example, a freezer.

According to the present embodiment, since cells, microorganisms, viruses, and dust present outside the cell culture vessel 22 do not enter to the closed cell culture vessel 22, the cleanliness in the cell culture vessel 22 is maintained. Therefore, the cell culture vessel 22 may not be disposed in the clean room. Carbon dioxide gas, nitrogen gas, oxygen gas and the like may or may not be supplied into the closed system in which cells exist. In the case where a gas is supplied into the closed system, for example, the gas may be supplied to the flow path or the like through a gas exchange filter, and a gas may be supplied into the cell culture vessel 22.

According to the cell culture device of the embodiment, for example, since cells are cultured in the completely closed system, it is possible to reduce a risk of cross-contamination due to leakage of cells from the culture device. In addition, for example, even if cells are infected with viruses such as HIV hepatitis viruses, it is possible to reduce a risk of infection with an operator due to cell leakage. In addition, it is possible to reduce a risk of the medium in the cell culture vessel contaminating the air outside the cell culture vessel with bacteria, viruses, molds and the like. In addition, according to the cell culture vessel of the embodiment, it is possible to culture cells without using a CO₂ incubator.

While the present invention has been described above with reference to the embodiment, the descriptions and drawings that form some of the disclosure should not be understood as limiting the invention. Those skilled in the art can clearly understand various alternative embodiments, embodiments and operational techniques from this disclosure. For example, the cells sent to the cell culture vessel 22 shown in Fig. 1 and Fig. 2 are not limited to the blood cells such as the mononuclear cells. The cells sent to the cell culture vessel 22 may be stem cells, fibroblasts, nerve cells, retinal epithelial cells, hepatocytes, β cells, renal cells, mesenchymal stem cells, blood cells, megakaryocytes, T cells, chondrocytes, cardiomyocytes, muscle cells, vascular cells, epithelial cells, pluripotent stem cells, ES cell, iPS cells, or other somatic cells. The cells sent to the cell culture vessel 22 are arbitrary.

In addition, while an example in which the iPS cells are produced from the mononuclear cells in the cell culture vessel 22 has been described in the embodiment, fibroblasts, nerve cells, retinal epithelial cells, hepatocytes, β cells, renal cells, mesenchymal stem cells, blood cells, megakaryocytes, T cells, chondrocytes, cardiomyocytes, muscle cells, vascular cells, epithelial cells, pluripotent stem cells, ES cells, iPS cells, or differentiated cells such as other somatic cells may be produced from stem cells in the cell culture vessel 22. The stem cells may be iPS cells, embryonic stem cells (ES cells), somatic stem cells or other artificially induced stem cells. In this case, for example, the first variable volume container 27 holds a differentiation-inducing factor. Here, cells may be cultured without inducing the cells in the cell culture vessel 22.

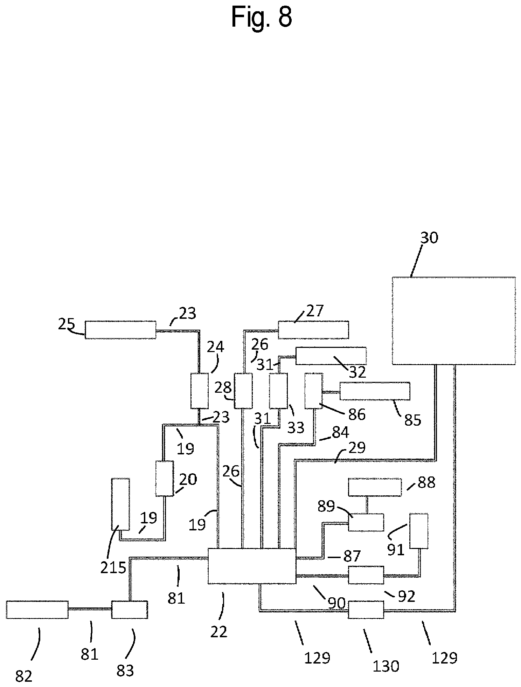

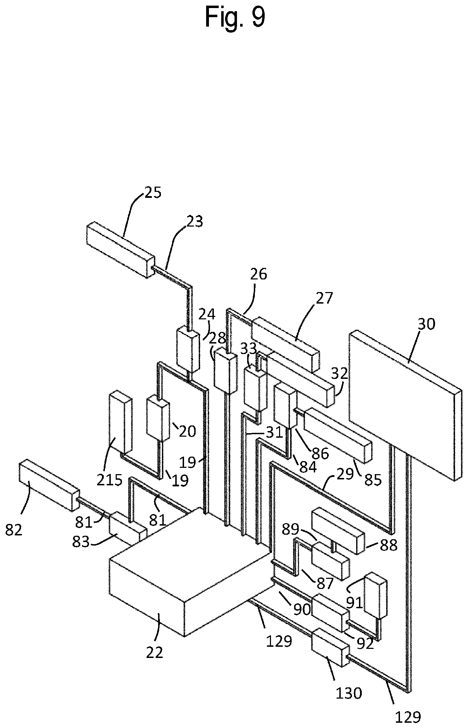

In addition, as described above, the cells prepared in advance may be supplied into the cell culture vessel 22. In this case, as shown in Fig. 8 and Fig. 9, the cells prepared in advance are held in a cell container 215, and the cells in the cell container 215 may be sent to the flow path 19. In this manner, it should be understood that the present invention includes various embodiments and the like.

### Examples

### (Example 1)

This example shows that cells could be cultured in a completely closed environment without medium replacement and gas exchange. A growth factor was added to a medium (StemSpan H3000, registered trademark, STEMCELL Technologies Inc.), and deacylated gellan gum was additionally added to the medium to prepare a gel medium.

The prepared gel medium was put into a 15 mL tube and 2×10⁵ blood cells were seeded in the gel medium. Then, the 15 mL tube was placed in a CO₂ incubator, and blood cells (mononuclear cells) were cultured for 7 days. Then, a Sendai virus vector harboring OCT3/4, SOX2, KLF4, and cMYC was added to the gel medium so that the multiplicity of infection (MOI) was 10.0, and the blood cells were infected with Sendai viruses.

After Sendai viruses were added to the gel medium, 15 mL of the gelled stem cell medium (DMEM/F12 containing 20%KnockOut SR (registered trademark, ThermoFisher SCIENTIFIC)) was added to the gel medium, and then 15 mL of the medium containing cells infected with the Sendai viruses was put into a sealable cell culture vessel, and the gel medium was injected into the cell culture vessel. Then, the interior of the cell culture vessel was sealed and gas exchange did not completely occur between the interior and the outside of the cell culture vessel.

Suspension culture of the cells into which the initialization factors were introduced was initiated in the cell culture vessel. Then, once every two days, 2 mL of the gel medium in a medium retention tank 40 was replaced with 2 mL of a new gel medium.

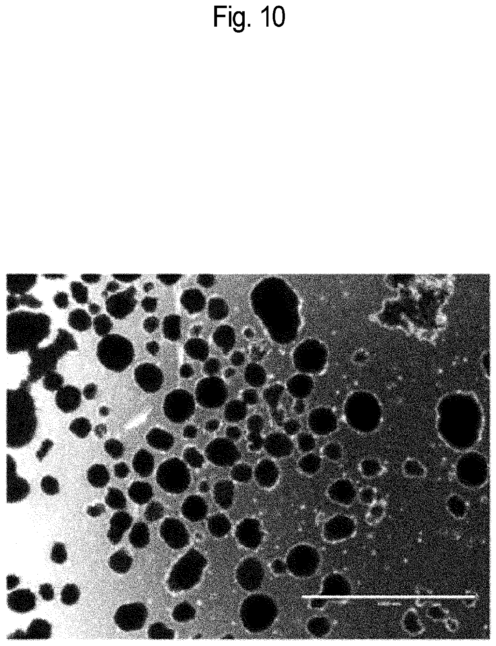

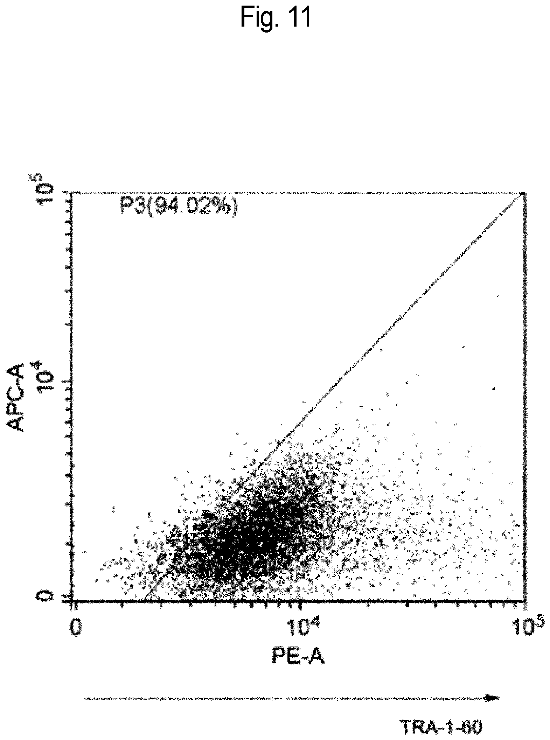

After 15 days, when the cells were observed under a microscope, as shown in Fig. 10, it was confirmed that ES cell-like colonies were formed. In addition, when cells were fixed using 4%-paraformaldehyde, and the expression level of cell surface antigen TRA-1-60 in the fixed cells was measured using a flow cytometer, as shown in Fig. 11, it was confirmed that more than 90% of the cells were TRA-1-60 positive, and the cells were almost completely reprogrammed. Therefore, in a completely closed environment, it was found that iPS cells can be induced from somatic cells other than stem cells without medium replacement and gas exchange.

### (Example 2)

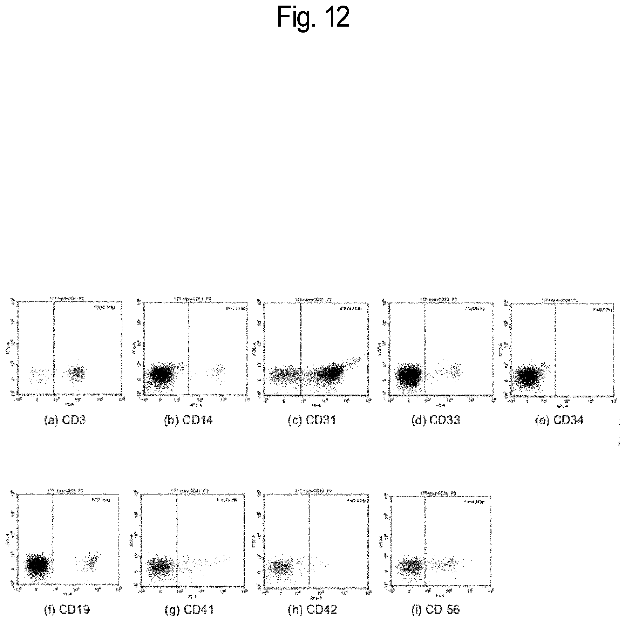

Blood was treated with a red blood cell precipitating agent to obtain treated blood from which red blood cells were at least partially removed. The treated blood was treated with surface cell marker antibodies and analyzed by fluorescence-activated cell sorting (FACS), and the results are shown in Fig. 12. The treated blood contained CD3 positive cells, CD14 positive cells, CD31 positive cells, CD33 positive cells, CD34 positive cells, CD19 positive cells, CD41 positive cells, CD42 positive cells, and CD56 positive cells.

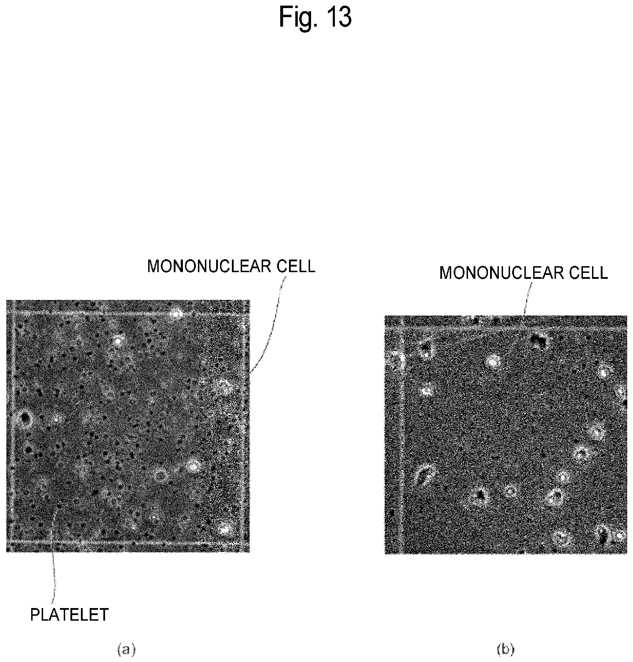

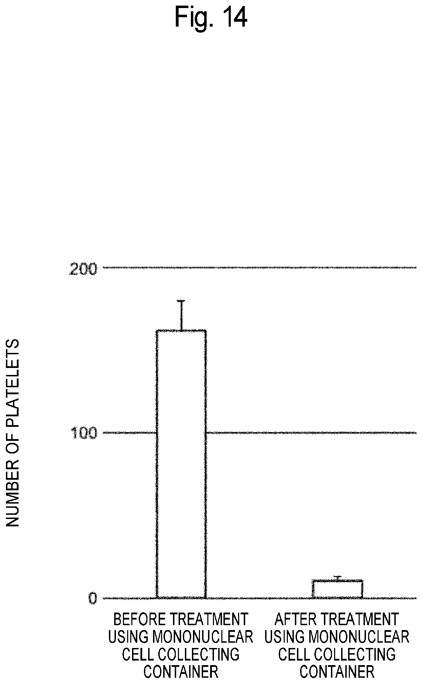

The treated blood from which the red blood cells were at least partially removed was put into a mononuclear cell collector as shown in Fig. 3, and diluted with a buffer solution, and the supernatant was removed. Then, mononuclear cells were collected from the mononuclear cell collector. As shown in Fig. 13(a), the treated blood before it was put into the mononuclear cell collector contained a large number of platelets. On the other hand, as shown in Fig. 13(b), the platelets were almost removed from the solution containing the mononuclear cells collected from the mononuclear cell collector. Fig. 14 shows a graph showing the number of platelets in the treated blood before it was put into the mononuclear cell collector and the number of platelets in the solution containing the mononuclear cells collected from the mononuclear cell collector in the same area.

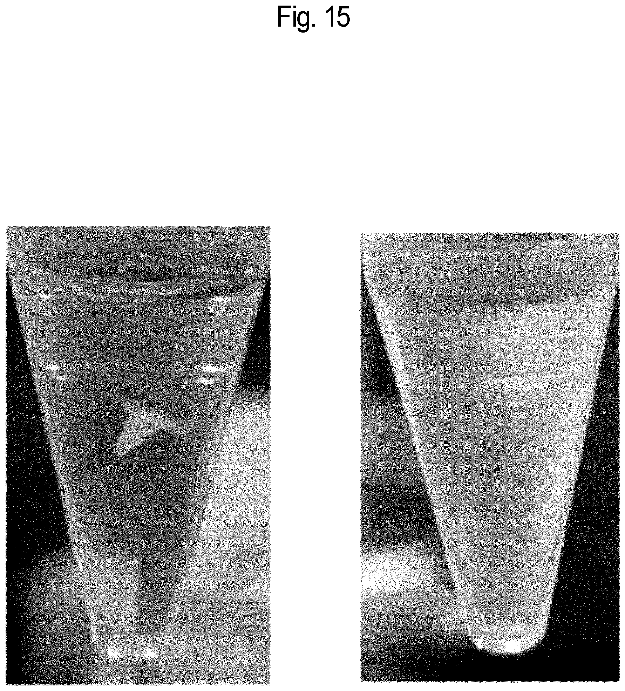

When the treated blood containing the platelets before it was put into the mononuclear cell collector was put into the culture solution, as shown in Fig. 15(a), aggregation occurred. On the other hand, when the solution containing the mononuclear cells from which the platelets had been removed, which were collected from the mononuclear cell collector, was put into the culture solution, as shown in Fig. 15(b), no aggregation occurred.

### (Example 3)

Deacylated gellan gum was added to a blood medium to prepare a gel medium. The prepared gel medium was put into a laminin-coated 6-well dish, and 2×10⁵ blood cells (mononuclear cells) were seeded. Then, the 6-well dish was placed in a CO₂ incubator at 37°C and the blood cells were cultured for 7 days. Then, Sendai virus vector (CytoTune-iPS2.0, ThermoFisher SCIENTIFIC) harboring OCT3/4, SOX2, KLF4, and cMYC was added to the blood growth medium so that the multiplicity of infection (MOI) was 5, and the blood cells were infected with Sendai viruses.

Two days after the Sendai viruses were added to the blood growth medium while the cells were put into a 6-well dish, and the medium was replaced using 500 µL of a stem cell medium (DMEM/F12 containing 20%KnockOut SR (registered trademark, ThermoFisher SCIENTIFIC)) or StemFit.

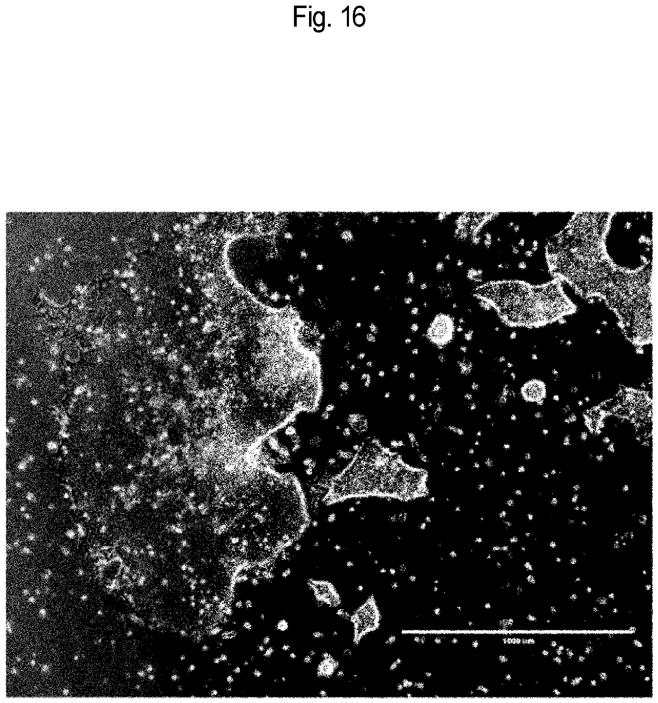

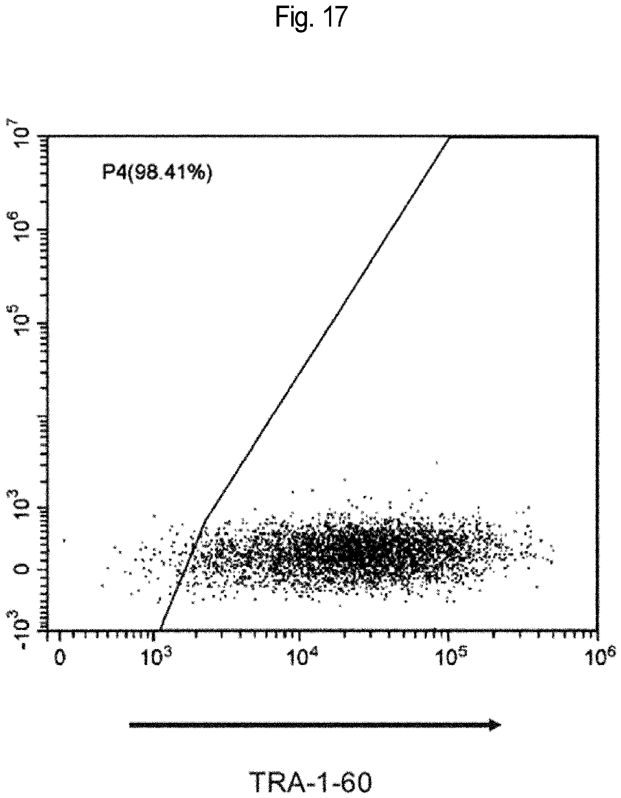

15 days after the Sendai viruses were added to the blood growth medium, when the cells were observed under a microscope, as shown in Fig. 16, it was confirmed that ES cell-like colonies were formed. In addition, when cells were fixed using 4%-paraformaldehyde, and the expression level of cell surface antigen TRA-1-60 in the fixed cells was measured using a flow cytometer, as shown in Fig. 17, it was confirmed that almost 100% of the cells after induction were TRA-1-60 positive, and the cells were almost completely reprogrammed. Therefore, it was found that, it is possible to reprogram cells by introducing reprogramming factors into the cells in a cell culture vessel and culturing the cells into which the reprogramming factors were introduced in the same cell culture vessel.

### (Example 4)

Deacylated gellan gum was added to a blood medium to prepare a gel medium. The prepared gel medium was put into a laminin-coated flask, and 5×10⁵ blood cells (mononuclear cells) were seeded, then, being placed in a CO₂ incubator at 37°C and the blood cells were cultured for 7 days. Then, Sendai virus vector (CytoTune-iPS2.0, ThermoFisher SCIENTIFIC) harboring OCT3/4, SOX2, KLF4, and cMYC was added to the blood growth medium so that the multiplicity of infection (MOI) was 5, and the blood cells were infected with Sendai viruses.

Two days after the Sendai viruses were added to the blood growth medium, the flask was completely filled with the stem cell medium (DMEM/F12 containing 20%KnockOut SR (registered trademark, ThermoFisher SCIENTIFIC)) or StemFit so that no air remained in the flask, the flask cap was closed to prevent exchange of a gas with the outside, and the interior of the flask was closed to prevent cells, microorganisms, impurities and the like from permeating.

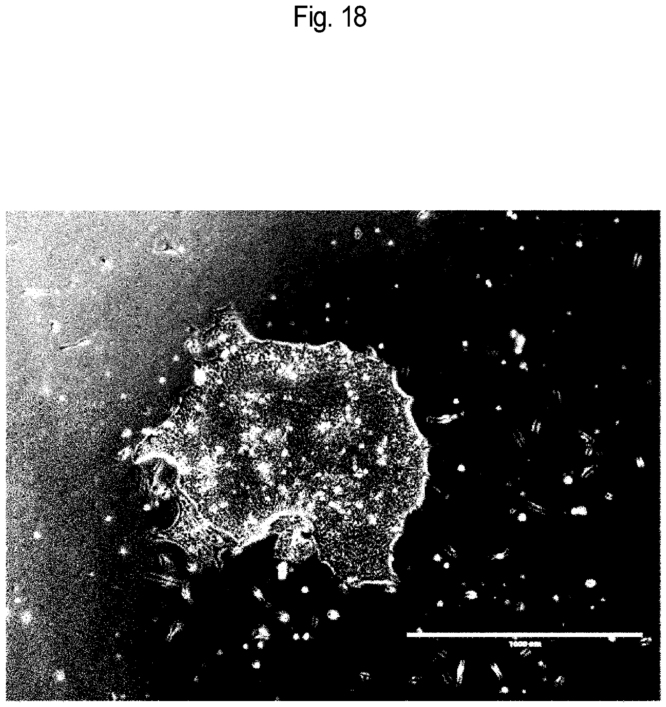

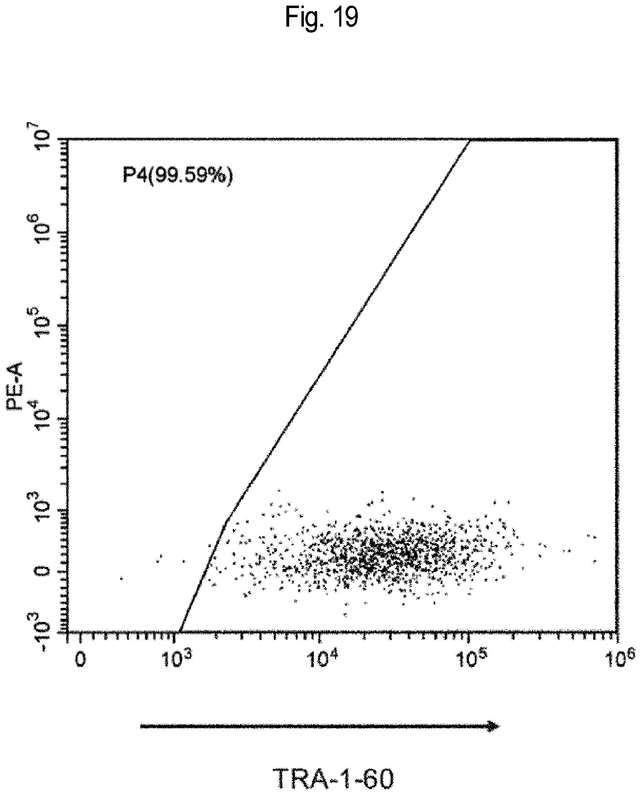

15 days after the Sendai viruses were added to the blood growth medium, when the cells were observed under a microscope, as shown in Fig. 18, it was confirmed that ES cell-like colonies were formed. In addition, when the cells were fixed using 4%-paraformaldehyde, and the expression level of cell surface antigen TRA-1-60 in the fixed cells was measured using a flow cytometer, as shown in Fig. 19, it was confirmed that almost 100% of the cells after induction were TRA-1-60 positive, and the cells were almost completely reprogrammed. Therefore, it was found that, it is possible to reprogram cells by introducing reprogramming factors into the cells in a cell culture vessel and culturing the cells into which the reprogramming factors were introduced in the same closed cell culture vessel.

### (Example 5)

A non-gelled liquid blood growth medium was put into a laminin-coated 6-well dish, and 2×10⁵ blood cells (mononuclear cells) were seeded. Then, the 6-well dish was placed in a CO₂ incubator at 37°C and the blood cells were cultured for 7 days. Then, Sendai virus vector (CytoTune-iPS2.0, ThermoFisher SCIENTIFIC) harboring OCT3/4, SOX2, KLF4, and cMYC was added to the blood growth medium so that the multiplicity of infection (MOI) was 5, and the blood cells were infected with Sendai viruses.

Two days after the Sendai viruses were added to the blood growth medium while the cells were put into a 6-well dish, and the medium was replaced using 500 µL of a stem cell medium (DMEM/F12 containing 20%KnockOut SR (registered trademark, ThermoFisher SCIENTIFIC)) or StemFit.

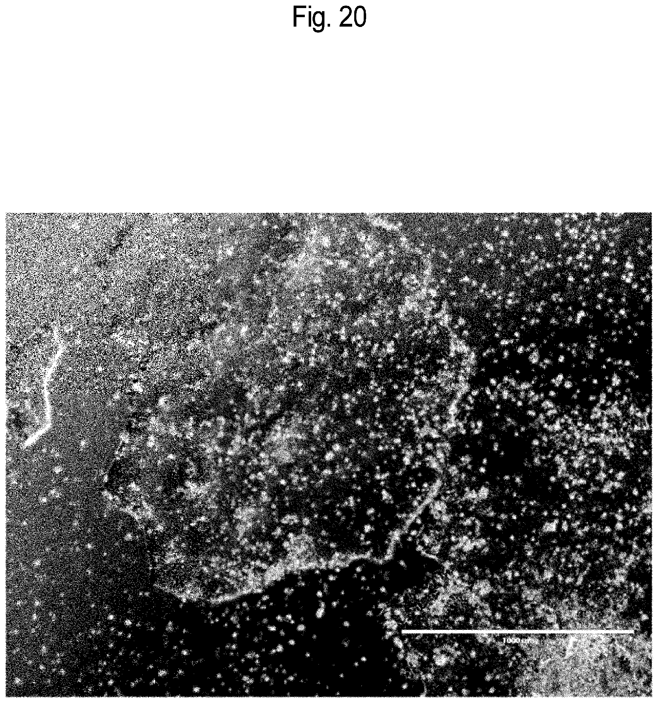

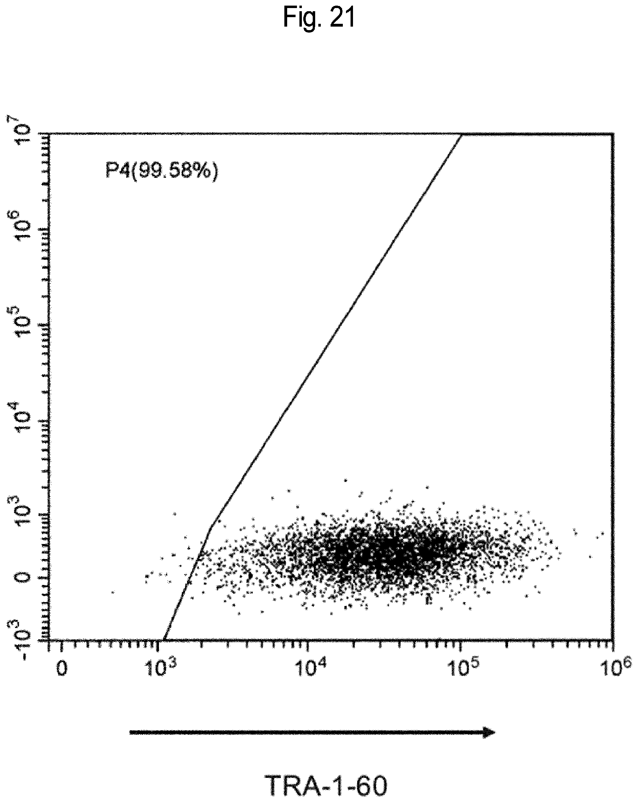

15 days after the Sendai viruses were added to the blood growth medium, when the cells were observed under a microscope, as shown in Fig. 20, it was confirmed that ES cell-like colonies were formed. In addition, when the cells were fixed using 4%-paraformaldehyde, and the expression level of cell surface antigen TRA-1-60 in the fixed cells was measured using a flow cytometer, as shown in Fig. 21, it was confirmed that almost 100% of the cells after induction were TRA-1-60 positive, and the cells were almost completely reprogrammed. Therefore, it was found that, it is possible to reprogram the cells by introducing reprogramming factors into the cells in a cell culture vessel and culturing the cells into which the reprogramming factors were introduced in the same cell culture vessel.

### (Example 6)

A non-gelled liquid blood growth medium was put into a laminin-coated flask, and 5×10⁵ blood cells (mononuclear cells) were seeded, then, being placed in a CO₂ incubator at 37°C and the blood cells were cultured for 7 days. Then, Sendai virus vector (CytoTune-iPS2.0, ThermoFisher SCIENTIFIC) harboring OCT3/4, SOX2, KLF4, and cMYC was added to the blood growth medium so that the multiplicity of infection (MOI) was 5, and the blood cells were infected with Sendai viruses.

Two days after the Sendai viruses were added to the blood growth medium, the flask was completely filled with the stem cell medium (DMEM/F12 containing 20%KnockOut SR (registered trademark, ThermoFisher SCIENTIFIC)) or StemFit so that no air remained in the flask, the flask cap was closed to prevent exchange of a gas with the outside, and the interior of the flask was closed to prevent cells, microorganisms, impurities and the like from permeating.

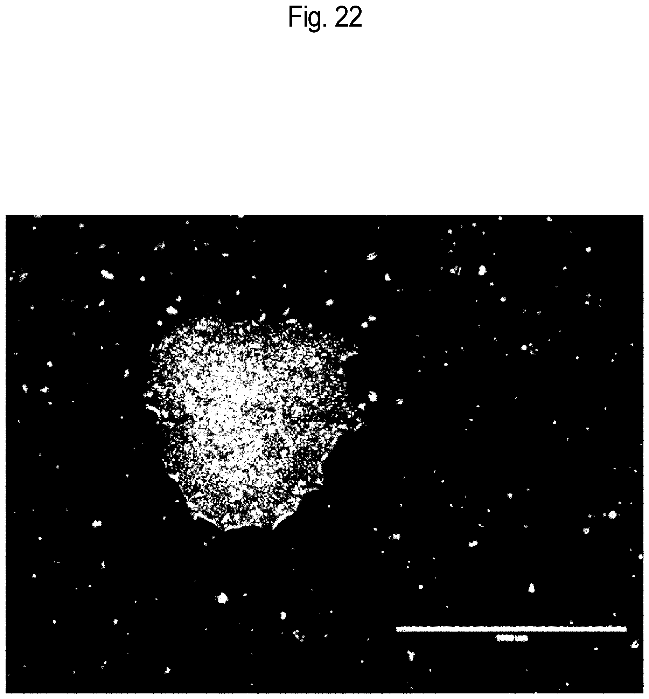

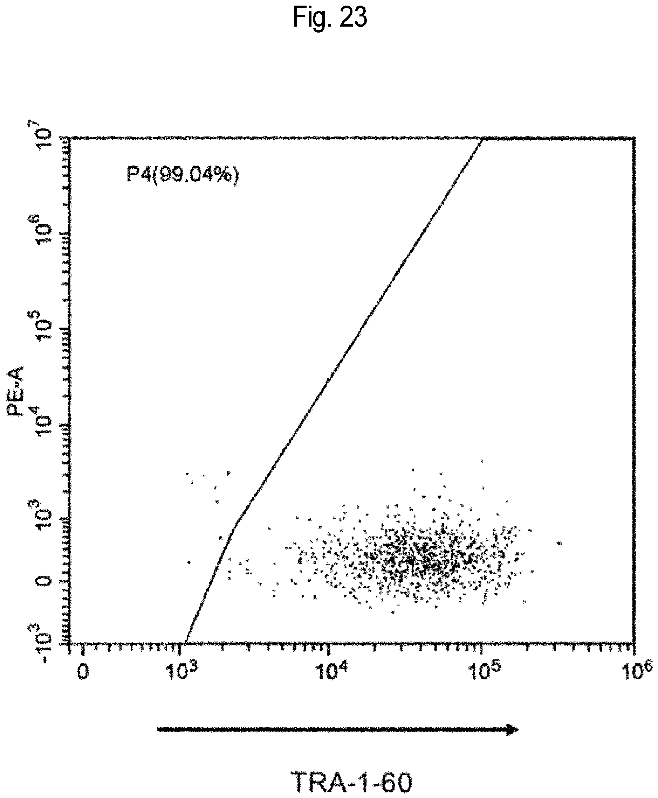

15 days after the Sendai viruses were added to the blood growth medium, when the cells were observed under a microscope, as shown in Fig. 22, it was confirmed that ES cell-like colonies were formed. In addition, when the cells were fixed using 4%-paraformaldehyde, and the expression level of cell surface antigen TRA-1-60 in the fixed cells was measured using a flow cytometer, as shown in Fig. 23, it was confirmed that almost 100% of the cells after induction were TRA-1-60 positive, and the cells were almost completely reprogrammed. Therefore, it was found that, it is possible to reprogram cells by introducing reprogramming factors into the cells in a cell culture vessel and culturing the cells into which the reprogramming factors were introduced in the same closed cell culture vessel.

### Reference Signs List

- 11: Red blood cell remover
- 15: Mononuclear cell collector
- 17: Flow path
- 18: Fluid machine
- 19: Flow path
- 20: Mononuclear cell aspiration device
- 21: Fluid machine
- 22: Cell culture vessel
- 23: Flow path
- 24: Fluid machine
- 25: Medium container
- 26: Flow path
- 27: Variable volume container
- 28: Fluid machine
- 29: Flow path
- 30: Variable volume container
- 31: Flow path
- 32: Medium container
- 33: Fluid machine
- 40: Medium retention tank
- 50: Blood container
- 51: Flow path
- 52: Fluid machine
- 53: Red blood cell treatment agent container
- 54: Flow path
- 55: Fluid machine
- 56: Flow path
- 57: Mixer
- 58: Flow path
- 60: Flow path
- 61: Diluting solution container
- 62: Fluid machine
- 81: Flow path
- 82: Coating agent container
- 83: Fluid machine
- 84: Flow path
- 85: Detachment solution container
- 86: Fluid machine
- 87: Flow path
- 88: Cryopreservation solution container
- 89: Fluid machine
- 90: Flow path
- 91: Cell cryopreservation container
- 92: Fluid machine
- 93: Flow path
- 115: Opening
- 116: Opening
- 117: Flow path
- 122: Medium component permeable member
- 123: Section
- 124: Section
- 129: Flow path
- 130: Fluid machine
- 215: Cell container
- 216: Flow path

## Claims

1. A cell culture vessel for culturing a cell in an interior thereof, wherein
a width of a bottom surface is equal to or larger than a height of a side surface,
the cell culture vessel comprises a flow path configured to supply a fluid into the interior, and
the interior is able to be closed.

2. A cell culture vessel for culturing a cell in an interior thereof, wherein
at least one of a bottom surface and a top surface is transparent,
the cell culture vessel comprises a flow path configured to supply a fluid into the interior, and
the interior is able to be closed.

3. The cell culture vessel according to claim 1 or 2, comprising:
a first housing having the bottom surface; and
a second housing which is disposed on the first housing and has a top surface that faces the bottom surface, wherein
the first housing and the second housing are combined to form the interior.

4. The cell culture vessel according to claim 3, wherein the flow path is provided at at least one of the first housing and the second housing.

5. The cell culture vessel according to any one of claims 1 to 4, further comprising a temperature adjuster configured to adjust a temperature in the cell culture vessel.

6. The cell culture vessel according to any one of claims 1 to 5, wherein an internal culture container is able to be disposed in the interior.

7. The cell culture vessel according to any one of claims 1 to 6, further comprising a medium component permeable member that is disposed in the interior.

8. A cell culture device, comprising:
a cell culture vessel for culturing a cell in an interior thereof; and
a variable volume container that is connected to the cell culture vessel, wherein
a width of a bottom surface of the cell culture vessel is equal to or larger than a height of a side surface,
the cell culture vessel comprises a flow path configured to supply a fluid into the interior,
the variable volume container is connected to the cell culture vessel via the flow path,
the fluid is movable in the cell culture vessel and the variable volume container, and
the interior of the cell culture vessel and the variable volume container is able to be closed.

9. A cell culture device, comprising:
a cell culture vessel for culturing a cell in an interior thereof; and
a variable volume container that is connected to the cell culture vessel, wherein
at least one of a bottom surface and a top surface is transparent,
the cell culture vessel comprises a flow path configured to supply a fluid into the interior,
the variable volume container is connected to the cell culture vessel via the flow path,
the fluid is movable in the cell culture vessel and the variable volume container, and
the interior of the cell culture vessel and the variable volume container is able to be closed.

10. The cell culture device according to claim 8 or 9, wherein the variable volume container is configured to hold a substance, and the substance comes into contact with the cell according to movement of the fluid.

11. The cell culture device according to any one of claims 8 to 10, further comprising a flow path configured to supply the cell into the cell culture vessel.

12. The cell culture device according to any one of claims 8 to 11, further comprising a fluid machine configured to supply the cell into the cell culture vessel.

13. The cell culture device according to any one of claims 8 to 12, further comprising a flow path configured to supply a medium into the cell culture vessel.

14. The cell culture device according to any one of claims 8 to 13, further comprising a flow path configured to supply a cell dissociation reagent into the cell culture vessel.

15. The cell culture device according to claim 14, further comprising a flow path configured to discharge at least part of cells detached from an inner surface of the cell culture vessel with the cell dissociation reagent to the outside of the cell culture vessel.

16. The cell culture device according to claim 14 or 15, wherein at least part of cells detached from the inner surface of the cell culture vessel with the cell dissociation reagent is returned to the cell culture vessel.

17. The cell culture device according to any one of claims 8 to 16, further comprising a flow path configured to supply a cell cryopreservation solution into the cell culture vessel.

18. The cell culture device according to any one of claims 8 to 17, further comprising a temperature adjuster configured to adjust a temperature in the cell culture vessel.

19. The cell culture device according to any one of claims 8 to 18, wherein an internal culture container is able to be disposed in the interior of the cell culture vessel.

20. The cell culture device according to any one of claims 8 to 19, further comprising a medium component permeable member that is disposed in the interior of the cell culture vessel.
